# FASCICULE DE BREVET EUROPEEN

(11) **EP 3 743 220 B1**
(45) Date de publication et mention de la délivrance du brevet: **09.03.2022**
(21) Numéro de dépôt: 19717507.8
(22) Date de dépôt: 24.01.2019
(51) Int. Cl.: A61M 15/00, B05B 11/00, B65D 83/54, C08G 63/183

(54) **DISPOSITIF DE DISTRIBUTION DE PRODUIT FLUIDE**
FLÜSSIGPRODUKTAUSGABEVORRICHTUNG
FLUID PRODUCT DISPENSING DEVICE

(30) Priorité: 24.01.2018 FR 1850540
(43) Date de publication de la demande: 02.12.2020
(73) Titulaire: APTAR France SAS, 27110 Le Neubourg (FR)
(72) Inventeur: LÉONÉ, Patrice, 27400 Acquigny (FR)
(74) Mandataire: CAPRI
(86) Numéro de dépôt international: PCT/FR2019/000011
(87) Numéro de publication internationale: WO 2019/145614

(56) Documents cités:
- EP-A1- 2 335 833
- EP-A1- 2 427 278
- EP-A1- 3 412 699
- FR-A1- 2 959 729

## Description

La présente invention concerne un dispositif de distribution de produit fluide.

Le domaine d'application privilégié d'un tel dispositif de distribution est notamment mais pas exclusivement la pharmaceutique.

Des distributeurs de produit fluide de l'art antérieur comportent généralement un organe de distribution, tel qu'une pompe ou une valve, en communication d'une part avec un ou plusieurs réservoir(s) de produit fluide et d'autre part avec un organe d'actionnement dudit organe de distribution. Certains distributeurs peuvent en outre comporter un compteur ou indicateur de doses, pour indiquer à l'utilisateur le nombre de doses distribuées ou restant à distribuer. Des parties de ces dispositifs de comptage sont souvent réalisées en matériaux plastiques, tels que notamment le PBT (polytéréphtalate de butylène), le POM (polyoxyméthylène), le PA (polyamide) ou le PE (polyéthylène). Or, l'utilisation de ces matériaux plastiques peut impliquer divers inconvénients. Ainsi, les coefficients de frottement, notamment pour le POM, ne sont pas optimaux et peuvent mener à un dysfonctionnement du dispositif. De plus, les propriétés mécaniques, notamment pour le POM, ne sont pas optimales, et peuvent également avoir un impact négatif sur l'actionnement du dispositif. Par ailleurs, les coûts de production, notamment pour des dispositifs utilisant les matériaux listés ci-dessus, peuvent s'avérer importants, en raison notamment de temps de cycle assez longs.

Les documents EP2335833, FR2959729 et EP2427278 décrivent des dispositifs de l'état de la technique.

La présente invention a pour but de surmonter les problèmes susmentionnés.

La présente invention a ainsi pour but de fournir un dispositif de distribution permettant un comptage fiable et reproductible à chaque actionnement.

La présente invention a également pour but de fournir un dispositif de distribution de produit fluide simple et peu coûteux à fabriquer.

La présente invention a donc pour objet un dispositif de distribution de produit fluide comprenant :
- au moins un réservoir apte à contenir du produit fluide à distribuer,
- un organe de distribution,
- un organe d'actionnement apte à actionner ledit organe de distribution,
- un orifice de distribution par lequel du produit fluide est distribué,
- un compteur ou indicateur de doses,
au moins une partie dudit compteur ou indicateur de doses étant réalisée en un matériau comprenant une polycétone aliphatique.

Avantageusement, ledit matériau comprend un terpolymère de polycétone aliphatique.

Avantageusement, ledit terpolymère est un terpolymère éthylène/propylène/monoxyde de carbone, ayant la formule :

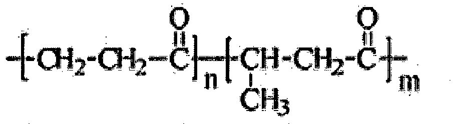

Selon un premier mode de réalisation avantageux, ledit organe de distribution est une valve, notamment une valve doseuse, contenant un gaz propulseur pour distribuer le produit fluide.

Avantageusement, le dispositif comprend un gaz HFA en tant que gaz propulseur.

Selon un second mode de réalisation avantageux, ledit organe de distribution est une pompe.

Avantageusement, ledit matériau est constitué de polycétone aliphatique.

En variante, ledit matériau est un alliage comprenant au moins une polycétone aliphatique et au moins un autre polymère.

Avantageusement, ledit au moins un autre polymère comprend un ou plusieurs des polymères suivants : le polyméthylméthacrylate (PMMA), le polybutylène téréphtalate (PBT), le polyacétal (POM), le polyéthylène glycol (PETG), le polyvinylchlorure (PVC), le polyamide (PA), le polycarbonate (PC), le polystyrène (PS), le styrène acrylonitrile (SAN), l'acrylonitrile butadiène styrène (ABS), le polyéthylène haute densité (PEHD), le polyéthylène basse densité (PEBD), l'alliage polysulfone (PSU), le polyéthylène téréphtalate (PET), l'élastomère thermoplastique polyuréthane (TPUR), le polyphénylène sulfure (PPS), le polyéthersulfone (PES), l'élastomère thermoplastique polyester (TPE), le polyphénylène éther oxide modifié (PPO), le polyétherimide (PEI), le polyétheréthercétone (PEEK), le polyuréthane thermoplastique rigide (RTPU), l'élastomère styrénique saturé (SEBS), l'élastomère styrénique insaturé (SBS), l'élastomère thermoplastique oléfinique (TEO), l'élastomère styrénique vulcanizè (TPV), le polyméthylpentène (PMP), le perfluoroalkoxy (PFA), l'éthylène térafluoroéthylène (ETFE), le polyvinylidène fluorure (PVDF), le polymère à cristaux liquides (LCP), l'éthylène propylène fluoré (FEP), le polyphtalamide (PPA), le polyéthercétonecétone (PEKK), le polyimide thermoplastique (TPI), le polyamide hautes températures (NHT), le polystyrène syndiotatique (SPS), le polytriméthylène téréphtalate (PTT).

Ces caractéristiques et avantages et d'autres de la présente invention apparaitront plus clairement au cours de la description détaillée suivante, faite en référence aux dessins joints, donnés à titre d'exemples non limitatifs, et sur lesquels:
La figure 1 illustre un exemple de dispositif de distribution,
La figure 2 illustre un autre exemple de dispositif de distribution,
La figure 3 illustre un exemple de dispositif de distribution auquel la présente invention s'applique,
La figure 4 illustre la résistance aux chocs pour trois matériaux différents,
La figure 5 illustre le coefficient de frottement sur du caoutchouc nitrile pour trois matériaux différents, et
La figure 6 illustre les niveaux d'extractibles pour deux matériaux différents.

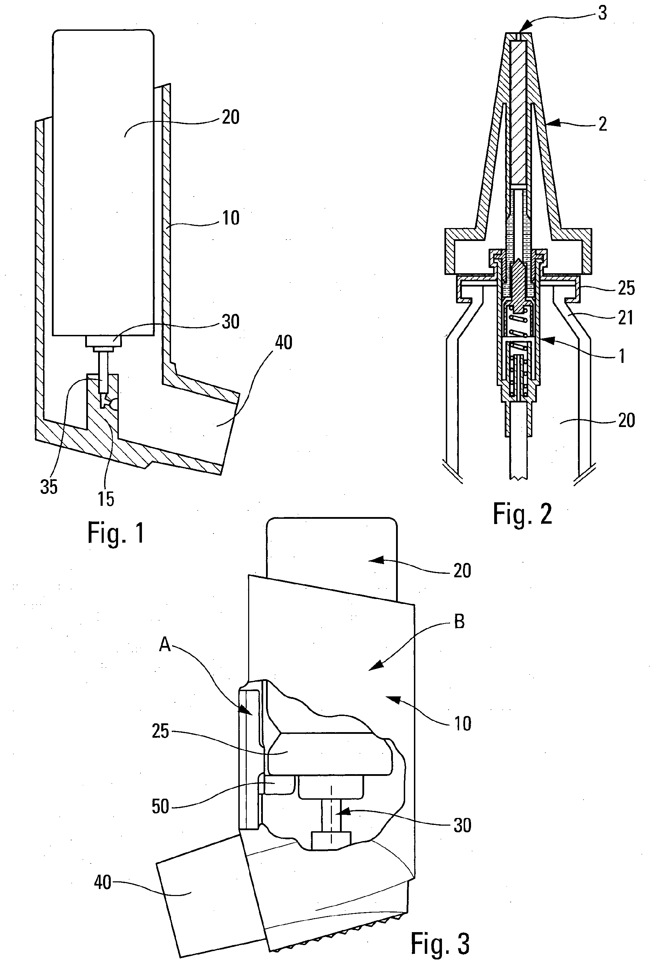

La présente invention s'applique à tous types de dispositifs de distribution de produit fluide comportant un dispositif de comptage de dose. En particulier, la présente invention peut s'appliquer à des distributeurs avec compteur comportant une valve, comme représenté sur la figure 1, ou une pompe, comme représenté sur la figure 2.

En se référant à la figure 1, il est décrit un inhalateur à dose mesurée, appelé généralement pMDI (« pressurized Metered Dose Inhaler »), qui classiquement comporte un corps 10 pourvu d'un orifice de distribution 40, généralement un embout buccal. A l'intérieur de ce corps est disposé un réservoir 20 sur lequel est montée une valve doseuse 30. Une soupape 35 coulisse dans le corps de valve de ladite valve doseuse 30 pour distribuer une dose de produit fluide à chaque actionnement. Le corps 10 comporte un puits de soupape 15 qui reçoit la soupape 35 et qui crée un passage de liaison entre la sortie de la soupape 35 et ledit orifice de distribution 40. De manière classique, pour actionner un tel dispositif, l'utilisateur appuie sur le fond du réservoir 20 pour enfoncer celui-ci axialement à l'intérieur du corps 10, ce qui provoque le coulissement étanche de la soupape 35 vers l'intérieur de la valve doseuse, provoquant ainsi la distribution d'une dose de produit fluide. A l'intérieur du réservoir, le produit fluide, qui contient généralement un ou plusieurs produit(s) actif(s), est associé à un gaz propulseur, de préférence un gaz du type HFA, par exemple HFA 134a et/ou HFA 227 et/ou HFA 152a. Généralement, on prévoit dans le réservoir 20, autour du corps de la valve doseuse 30, une bague dite bague fin de bidon (non représentée), notamment pour limiter le volume mort dans le réservoir.

Typiquement, dans ce type de dispositif, les corps de valve sont réalisés en PBT ou POM, la soupape est réalisée en POM ou PBT, et la bague est réalisée en PA ou PE.

En référence à la figure 2, le dispositif de distribution comporte une pompe 1 fixée sur le col 21 d'un récipient 20, par exemple au moyen d'une bague de fixation 25. Le dispositif comporte en outre une tête de distribution 2. Cette tête de distribution 2 comporte un canal d'expulsion reliant la pompe 1 à un orifice de distribution 3 de produit. Avantageusement, dans l'exemple représenté sur la figure 2, la tête de distribution 2 est un poussoir nasal. Bien entendu, l'invention s'applique à tout autre type quelconque de pompe et/ou de poussoir.

Typiquement, dans ce type de dispositif, les corps de pompe sont réalisés en PBT ou POM, et le piston en POM.

La figure 3 représente schématiquement un dispositif de distribution B pourvu d'un indicateur de doses A. Ce dispositif B comporte un corps 10 et un réservoir 20 sur lequel est assemblé une valve doseuse 30 au moyen d'une bague de fixation 25, telle qu'une capsule sertissable. L'actionnement du dispositif B étant obtenu par déplacement axial du réservoir 20 à l'intérieur du corps 10, ce déplacement entraînant une compression de la soupape de la valve 30 ce qui provoque l'expulsion d'une dose de produit à travers un orifice buccal 40, ainsi que l'actionnement de l'indicateur de dose A, par déplacement axial d'un actionneur 50. Bien entendu, il ne s'agit ici que d'un exemple de réalisation, et la présente invention pourrait s'adapter à tous types de compteurs ou indicateurs de doses, qu'ils soient associés à des valves ou à des pompes.

Typiquement, dans ce type de dispositif d'indicateur ou compteur de doses, l'actionneur est généralement réalisé en POM.

Selon l'invention, au moins une partie du compteur ou indicateur de dose A est réalisée en un matériau comprenant une polycétone aliphatique.

Les polycétones sont des thermoplastiques hautes performances, ayant la formule suivante :

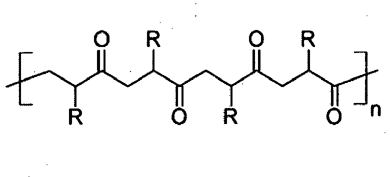

Les polycétones se divisent en 2 familles : les polycétones aliphatiques, appelées aussi POK, et les polycétones aromatiques, tel que la polyétheréthercétone, plus connue sous le nom de PEEK.

Les polycétones aliphatiques sont apparues dans les années 1990 puis ont été arrêtées en 2000 à cause des difficultés de mise en œuvre. La société coréenne Hyosung les a relancés en 2013. Elle a notamment développé des terpolymères (éthylène, propylène, CO) qui permettent d'avoir une meilleure processabilité ou aptitude à être travaillé (notamment une température de fusion plus basse) :

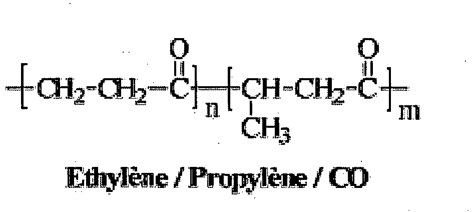

La présence de groupement carbonyle dans la chaine principale de leur structure chimique leur confère des propriétés intéressantes telles que :
- une bonne résistance à l'usure ;
- un bonne tenue à l'hydrolyse ;
- un niveau élevé de propriétés mécaniques ;
- des temps de cycle de moulage courts.

Les applications habituelles des polycétones se situent dans l'industrie pétrolière. Il peut notamment être intéressant de les utiliser pour limiter les migrations de produits chimiques dans les systèmes de transport ou pour limiter la corrosion dans ces systèmes de transport. Dans l'industrie automobile, ils peuvent être utilisés pour des connecteurs où il est nécessaire d'avoir sous le capot moteur une bonne tenue thermique et une bonne résistance aux carburants. Dans le bâtiment, sous des climats avec de fortes amplitudes thermiques, on peut remplacer avantageusement le nylon chargé de fibres de verre par du polycétone chargé de fibres de verre.

Les polycétones, notamment les polycétones aliphatiques, n'ont jamais été utilisés en tant que matériau pour réaliser des parties de compteur ou indicateur de doses.

Pour ce type d'application, il n'est pas nécessaire d'avoir une bonne étanchéité aux carburants, ni des résistances thermiques élevées. En revanche, la résistance mécanique des polycétones et les frottements réduits s'avèrent intéressants pour des composants de compteurs.

Par rapport aux matériaux habituellement utilisés listés ci-dessus, les polycétones aliphatiques présentent notamment les avantages suivants :
- résolution de la problématique du formaldéhyde liée au POM ;
- coefficients de frottement améliorés par rapport au PBT ;
- meilleures propriétés mécaniques que le PA ;
- gains économiques de production par des temps de cycle plus courts.

### Propriétés mécaniques :

Un des tests de caractérisation des propriétés mécaniques d'un matériau consiste à mesurer sa résistance au choc. Le principe consiste à déterminer l'énergie nécessaire pour rompre en une seule fois une éprouvette préalablement entaillée ou pas. Cette énergie nécessaire à la rupture est obtenue en calculant la différence de potentiels du marteau en position de départ (position la plus haute) et la position d'arrivée après rupture de l'éprouvette.

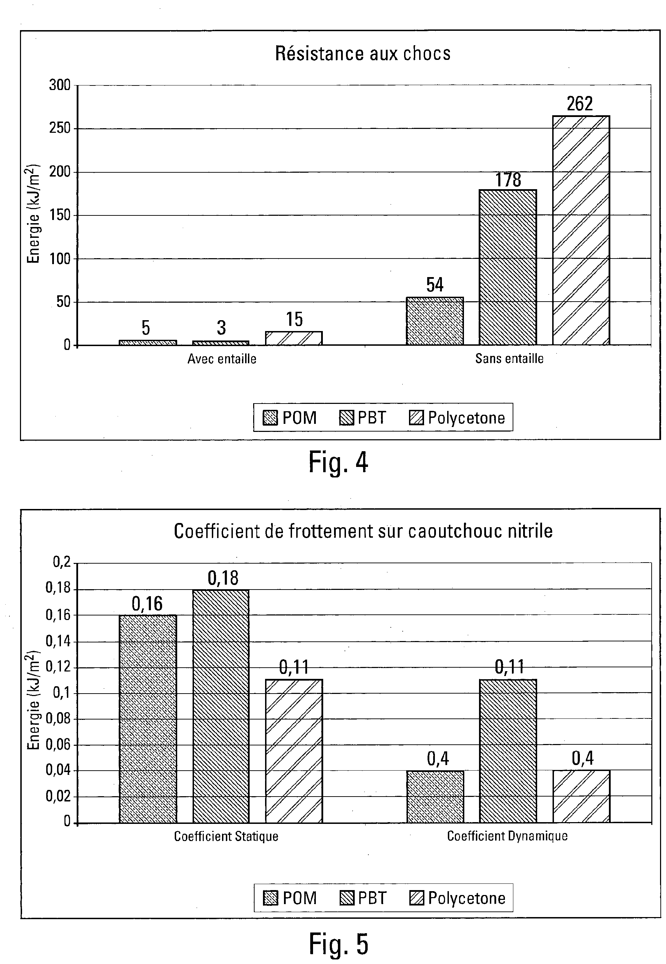

Dans le diagramme de la figure 4, on constate une nette amélioration de la résistance au choc avec les polycétone, par rapport au PBT et au POM.

### Frottement :

Le test consiste à faire frotter deux matériaux l'un sur l'autre afin de déterminer un coefficient de frottement. Le matériau utilisé pour réaliser ce test comparatif est le caoutchouc nitrile.

Le coefficient de frottement est le rapport de la force de traction (force de réponse permettant la mise en mouvement de l'appareil) sur la force appliquée (force normale).

Il existe deux types de coefficients : un dynamique et un statique.
- Le coefficient statique est le coefficient mesuré en début de test ; c'est la force nécessaire pour déplacer l'échantillon sur le substrat et initier le mouvement ; on parle aussi de coefficient d'adhérence ;
- Le coefficient dynamique est le coefficient nécessaire pour que le mouvement soit maintenu à une vitesse constante autrement.

Les résultats obtenus, illustrés sur la figure 5, montrent que les polycétones aliphatiques ont :
- un coefficient de frottement statique plus faible que le PBT et le POM ;
- un coefficient de frottement dynamique plus faible que le PBT et du même niveau que le POM.

### Extractibles :

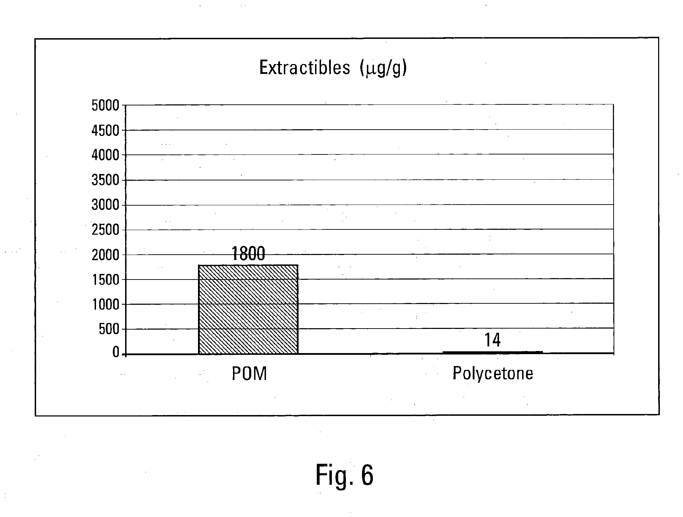

La figure 6 illustre que les niveaux d'extractibles mesurées pour les polycétones aliphatiques sont bien inférieurs à ceux du POM.

Il est possible de réaliser les parties du compteur ou indicateur de dose avec un matériau comprenant un alliage d'au moins une polycétone aliphatique avec au moins un autre polymère. Pour former de tels alliages, ledit au moins un autre polymère peut être choisi parmi les polymères suivants : le polyméthylméthacrylate (PMMA), le polybutylène téréphtalate (PBT), le polyacétal (POM), le polyéthylène glycol (PETG), le polyvinylchlorure (PVC), le polyamide (PA), le polycarbonate (PC), le polystyrène (PS), le styrène acrylonitrile (SAN), l'acrylonitrile butadiène styrène (ABS), le polyéthylène haute densité (PEHD), le polyéthylène basse densité (PEBD), l'alliage polysulfone (PSU), le polyéthylène téréphtalate (PET), l'élastomère thermoplastique polyuréthane (TPUR), le polyphénylène sulfure (PPS), le polyéthersulfone (PES), l'élastomère thermoplastique polyester (TPE), le polyphénylène éther oxide modifié (PPO), le polyétherimide (PEI), le polyétheréthercétone (PEEK), le polyuréthane thermoplastique rigide (RTPU), l'élastomère styrénique saturé (SEBS), l'élastomère styrénique insaturé (SBS), l'élastomère thermoplastique oléfinique (TEO), l'élastomère styrénique vulcanizè (TPV), le polyméthylpentène (PMP), le perfluoroalkoxy (PFA), l'éthylène térafluoroéthylène (ETFE), le polyvinylidène fluorure (PVDF), le polymère à cristaux liquides (LCP), l'éthylène propylène fluoré (FEP), le polyphtalamide (PPA), le polyéthercétonecétone (PEKK), le polyimide thermoplastique (TPI), le polyamide hautes températures (NHT), le polystyrène syndiotatique (SPS), le polytriméthylène téréphtalate (PTT). Toutefois, cette liste de polymères ne doit pas être considérée comme étant limitative, tous polymères aptes à être combinés audit au moins un polycétone aliphatique pouvant être utilisés.

Par conséquent, la présente invention propose une solution avantageuse et efficace destinée à optimiser les propriétés du matériau. Cette invention est donc notamment, mais pas exclusivement, particulièrement intéressante pour la distribution de formulations pharmaceutiques.

La présente invention a été décrite en référence à plusieurs modes de réalisation avantageux, mais il est entendu qu'un homme du métier peut y apporter toutes modifications, sans sortir du cadre de la présente invention tel que défini par les revendications annexées.

## Revendications

1. Dispositif de distribution de produit fluide comprenant :
- au moins un réservoir (20) apte à contenir du produit fluide à distribuer,
- un organe de distribution (1 ; 30),
- un organe d'actionnement (2 ; 10) apte à actionner ledit organe de distribution (1 ; 30),
- un orifice de distribution (3 ; 40) par lequel du produit fluide est distribué,
- un compteur ou indicateur de doses (A),
**caractérisé en ce qu'**au moins une partie dudit compteur ou indicateur de doses (A) est réalisée en un matériau comprenant une polycétone aliphatique.

2. Dispositif selon la revendication 1, dans lequel ledit matériau comprend un terpolymère de polycétone aliphatique.

3. Dispositif selon la revendication 2, dans lequel ledit terpolymère est un terpolymère éthylène/propylène/monoxyde de carbone, ayant la formule :

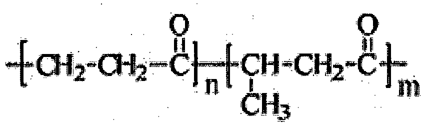

4. Dispositif selon l'une quelconque des revendications précédentes, dans lequel ledit organe de distribution est une valve (30), notamment une valve doseuse, contenant un gaz propulseur pour distribuer le produit fluide.

5. Dispositif selon la revendication 4, comprenant un gaz HFA en tant que gaz propulseur.

6. Dispositif selon l'une quelconque des revendications 1 à 3, dans lequel ledit organe de distribution est une pompe (1).

7. Dispositif selon l'une quelconque des revendications précédentes, dans lequel ledit matériau est constitué de polycétone aliphatique.

8. Dispositif selon l'une quelconque des revendications 1 à 6, dans lequel ledit matériau est un alliage comprenant au moins une polycétone aliphatique et au moins un autre polymère.

9. Dispositif selon la revendication 8, dans lequel ledit au moins un autre polymère comprend un ou plusieurs des polymères suivants : le polyméthylméthacrylate (PMMA), le polybutylène téréphtalate (PBT), le polyacétal (POM), le polyéthylène glycol (PETG), le polyvinylchlorure (PVC), le polyamide (PA), le polycarbonate (PC), le polystyrène (PS), le styrène acrylonitrile (SAN), l'acrylonitrile butadiène styrène (ABS), le polyéthylène haute densité (PEHD), le polyéthylène basse densité (PEBD), l'alliage polysulfone (PSU), le polyéthylène téréphtalate (PET), l'élastomère thermoplastique polyuréthane (TPUR), le polyphénylène sulfure (PPS), le polyéthersulfone (PES), l'élastomère thermoplastique polyester (TPE), le polyphénylène éther oxide modifié (PPO), le polyétherimide (PEI), le polyétheréthercétone (PEEK), le polyuréthane thermoplastique rigide (RTPU), l'élastomère styrénique saturé (SEBS), l'élastomère styrénique insaturé (SBS), l'élastomère thermoplastique oléfinique (TEO), l'élastomère styrénique vulcanizè (TPV), le polyméthylpentène (PMP), le perfluoroalkoxy (PFA), l'éthylène térafluoroéthylène (ETFE), le polyvinylidène fluorure (PVDF), le polymère à cristaux liquides (LCP), l'éthylène propylène fluoré (FEP), le polyphtalamide (PPA), le polyéthercétonecétone (PEKK), le polyimide thermoplastique (TPI), le polyamide hautes températures (NHT), le polystyrène syndiotatique (SPS), le polytriméthylène téréphtalate (PTT).

## Patentansprüche

1. Vorrichtung zur Ausgabe eines Fluidprodukts, enthaltend:
- zumindest einen Behälter (20), der dazu geeignet ist, das auszugebende Fluidprodukt zu enthalten,
- ein Ausgabeorgan (1; 30),
- ein Betätigungsorgan (2; 10), das dazu geeignet ist, das Ausgabeorgan (1; 30) zu betätigen,
- eine Ausgabeöffnung (3; 40), durch die Fluidprodukt ausgegeben wird,
- einen Dosierungszähler oder -anzeiger (A),
**dadurch gekennzeichnet, dass** zumindest ein Teil des Dosierungszählers oder -anzeigers (A) aus einem Material hergestellt ist, das ein aliphatisches Polyketon umfasst.

2. Vorrichtung nach Anspruch 1, wobei das Material ein Terpolymer eines aliphatischen Polyketons umfasst.

3. Vorrichtung nach Anspruch 2, wobei das Terpolymer ein Ethylen/Propylen/Kohlenmonoxid-Terpolymer ist mit der Formel:

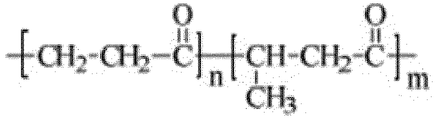

4. Vorrichtung nach einem der vorhergehenden Ansprüche, wobei das Ausgabeorgan ein Ventil (30), insbesondere ein Dosierventil, ist, das ein Treibgas enthält, um das Fluidprodukt auszugeben.

5. Vorrichtung nach Anspruch 4, enthaltend ein HFA-Gas als Treibgas.

6. Vorrichtung nach einem der Ansprüche 1 bis 3, wobei das Ausgabeorgan eine Pumpe (1) ist.

7. Vorrichtung nach einem der vorhergehenden Ansprüche, wobei das Material aus aliphatischem Polyketon besteht.

8. Vorrichtung nach einem der Ansprüche 1 bis 6, wobei das Material eine Legierung ist, die zumindest ein aliphatisches Polyketon und zumindest ein weiteres Polymer enthält.

9. Vorrichtung nach Anspruch 8, wobei das zumindest eine weitere Polymer eines oder mehrere der folgenden Polymere umfasst: Polymethylmethacrylat (PMMA), Polybutylenterephthalat (PBT), Polyacetal (POM), Polyethylenglykol (PETG), Polyvinylchlorid (PVC), Polyamid (PA), Polycarbonat (PC), Polystyrol (PS), Styrolacrylnitril (SAN), Acrylnitrilbutadienstyrol (ABS), Polyethylen hoher Dichte (PE-HD), Polyethylen niedriger Dichte (PE-LD), Polysulfonlegierung (PSU), Polyethylenterephthalat (PET), thermoplastisches Polyurethanelastomer (TPUR), Polyphenylensulfid (PPS), Polyethersulfon (PES), thermoplastisches Polyesterelastomer (TPE), modifiziertes Polyphenylenetheroxid (PPO), Polyetherimid (PEI), Polyetheretherketon (PEEK), steifes thermoplastisches Polyurethan (RTPU), gesättigtes Styrol-Elastomer (SEBS), ungesättigtes Styrol-Elastomer (SBS), olefinisches thermoplastisches Elastomer (TEO), vulkanisiertes Styrol-Elastomer (TPV), Polymethylpenten (PMP), Perfluoralkoxy (PFA), Ethylen-Terafluorethylen (ETFE), Polyvinylidenfluorid (PVDF), Flüssigkristallpolymer (LCP), fluoriertes Ethylenpropylen (FEP), Polyphthalamid (PPA), Polyetherketonketon (PEKK), thermoplastisches Polyimid (TPI), Hochtemperaturpolyamid (NHT), syndiotaktisches Polystyrol (SPS), Polytrimethylenterephthalat (PTT).

## Claims

1. A fluid dispenser device comprising:
• at least one reservoir (20) that is suitable for containing fluid to be dispensed,
• a dispenser member (1; 30),
• an actuator member (2; 10) that is suitable for actuating said dispenser member (1; 30),
• a dispenser orifice (3; 40) via which the fluid is dispensed,
• a dose counter or indicator (A),
**characterized in that** at least a portion of said dose counter or indicator (A) is made out of a material comprising an aliphatic polyketone.

2. A device according to claim 1, wherein said material comprises an aliphatic polyketone terpolymer.

3. A device according to claim 2, wherein said terpolymer is an ethylene/propylene/carbon monoxide terpolymer, having the formula:

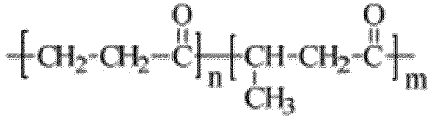

4. A device according to any preceding claim, wherein said dispenser member is a valve (30), in particular a metering valve containing a propellant gas for dispensing the fluid.

5. A device according to claim 4, containing an HFA gas as a propellant gas.

6. A device according to any one of claims 1 to 3, wherein said dispenser member is a pump (1).

7. A device according to any preceding claim, wherein said material is constituted by aliphatic polyketone.

8. A device according to any one of claims 1 to 6, wherein said material is an alloy comprising at least one aliphatic polyketone and at least one other polymer.

9. A device according to claim 8, wherein said at least one other polymer comprises one or more of the following polymers: polymethyl methacrylate (PMMA), polybutyl terephthalate (PBT), polyacetal (POM), polyethylene glycol (PETG), polyvinyl chloride (PVC), polyamide (PA), polycarbonate (PC), polystyrene (PS), styrene acrylonitrile (SAN), acrylonitrile butadiene styrene (ABS), high-density polyethylene (HDPE), low-density polyethylene (LDPE), polysulfone (PSU) alloy, polyethylene terephthalate (PET), thermoplastic polyurethane (TPUR) elastomer, polyphenylene sulfide (PPS), polyethersulfone (PES), thermoplastic polyester elastomer (TPE), modified polyphenylene oxide (PPO), polyetherimide (PEI), polyetheretherketone (PEEK), rigid thermoplastic polyurethane (RTPU), saturated styrenic elastomer (SEBS), unsaturated styrenic elastomer (SBS), olefinic thermoplastic elastomer (TEO), vulcanized styrenic elastomer (TPV), polymethylpentene (PMP), perfluoroalkoxy (PFA), ethylene tetrafluoroethylene (ETFE), polyvinylidene fluoride (PVDF), liquid crystal polymer (LCP), fluorinated ethylene propylene (FEP), polyphtalamide (PPA), polyetherketoneketone (PEKK), thermoplastic polyimide (TPI), high-temperature polyamide (NHT), syndiotactic polystyrene (SPS), polytrimethylene terephthalate (PTT).
